# EUROPEAN PATENT SPECIFICATION

(11) **EP 3 787 723 B1**
(45) Date of publication and mention of the grant of the patent: **28.02.2024**
(21) Application number: 19724259.7
(22) Date of filing: 28.04.2019
(51) Int. Cl.: A61M 16/06, A61M 16/12, A61M 16/08

(54) **NON-OBSTRUCTIVE NASAL CANNULA**
NICHTOBSTRUKTIVE NASENKANÜLE
CANULE NASALE NON OBSTRUCTIVE

(30) Priority: 29.04.2018 US 201862664176 P; 22.05.2018 US 201862674648 P
(43) Date of publication of application: 10.03.2021
(73) Proprietor: Sobel, Noam, 6811540 Jaffa (IL)
(72) Inventor: SOBEL, Noam, 6811540 Jaffa (IL); LIVNE, Ethan, 7610002 Rehovot (IL); WEISSBROD, Aharon, 7610002 Rehovot (IL); SHUSHAN, Sagit, 7610002 Rehovot (IL); HONIGSTEIN, Danielle Ricca, 7610002 Rehovot (IL)
(74) Representative: Dennemeyer & Associates S.A.
(86) International application number: PCT/IL2019/050470
(87) International publication number: WO 2019/211834

(56) References cited:
- WO-A1-2014/091362
- FR-A1- 2 827 778
- US-A1- 2005 121 033
- US-A1- 2015 000 661

## Description

### RELATED APPLICATION/S

This application claims the benefit of priority from U.S. Provisional Patent Application No. 62/664,176 filed on April 29, 2018 and from U.S. Provisional Patent Application No. 62/674,648 filed on May 22, 2018.

### FIELD AND BACKGROUND OF THE INVENTION

The present invention, in some embodiments thereof, relates to a nasal cannula and, more particularly, but not exclusively, to a structure for a nasal cannula that does not obstruct the nasal passage.

A nasal cannula is a device used to supply gases directly to a patient's nose and is also used to measure gases from a patients nose. The gas may be supplemental oxygen or increased airflow and may be supplied to a patient or person in need of respiratory help. The nasal cannula is formed with a tube that is split into two prongs which are placed in the nostrils and through which the supply gas flows into the nostrils. The tube may be held in place with a hook around the patient's ears or with an elastic head band. A distal end of the tube may be connected to a gas supply source such as a portable oxygen generator or central supply source in a hospital via a wall connection. It is also known to use a nasal cannula for infants or neonates. Gas flow rate that may be achieved with a nasal cannula for infants or neonates may be limited due to the small dimensions of the nostrils. However, higher rates may be achieved with a known wider channel humidified nasal cannula. Another known drawback of nasal cannulae for infants or neonates is that due to the small dimensions of the nostrils, the prongs may plug the nostril and so that there is little or no free airflow passage between the nostril and the surrounding environment. This may cause significant side effects such as positive end-distending pressure to infants and significant alteration to their breathing strategy.

French Patent Application Publication No. FR 2827778, entitled "Nasal respiratory assistance device," discloses a nasal apparatus for respiratory assistance. The apparatus includes two parallel tubular passages integral with one another and including distal ends that are respectively intended to be introduced into the two nostrils of a patient. Each of said tubular passages include at least one auxiliary channel opening into the corresponding tubular passage and with deflection means arranged opposite the corresponding auxiliary channel. The auxiliary channels of the two tubular passages are supplied in parallel with ventilation gas.

### SUMMARY OF THE INVENTION

The invention is defined in claim 1 and its appended dependent claims.

According to aspects of some example embodiments, there is provided a nasal cannula that supplies gases to a patient's nose without obstructing the nasal passage and the course of natural breathing through the nose. The nasal cannula may be particularly suitable for neonates, infants and small children. The nasal cannula as described herein enables a patient, e.g. a neonate and an adult patient to freely exhale through their nose and also to supplement inhalation of gases delivered with the nasal cannula with air from the surrounding environment. In some example embodiments, the nasal cannula is configured for sampling gases from the nasal cavity while the patient is breathing. Optionally, the nasal cannula is also configured for measuring changes in pressure in the nasal cavity during the breathing rhythm, and in this way measure breathing by providing a respiratory trace.

According to an aspect of some example embodiments, there is provided a nasal cannula comprising: a prong formed from an inner cylindrical wall surrounded by an outer cylindrical wall, a base connecting the inner cylindrical wall to the outer cylindrical wall, wherein the inner cylindrical wall, the outer cylindrical wall and base together define an open air channel between the inner and outer cylindrical wall and wherein the inner cylindrical wall defines a bore through which air freely flows; and a connector fluidly connected to the air channel and configured to provide flow communication between the air channel and an external device connected to the connector.

Optionally, the inner cylindrical wall has a first height and wherein the outer cylindrical wall has a second height and wherein the first height is less than the second height.

Optionally, the outer cylindrical wall has a truncated cone shape.

Optionally, the inner cylindrical wall has a first diameter and wherein the outer cylindrical wall has a second diameter at its distal end with respect to the base and wherein a ratio of the first diameter over the second diameter is 0.8-1.0.

Optionally, a width of the air channel tapers distal to the base.

Optionally, the connector is connected to the air channel via a port formed in the outer cylindrical wall.

Optionally, the base is configured to structurally support the prong and the connector and wherein the bore defined by the inner cylindrical wall extends through the base.

Optionally, the inner cylindrical wall is configured to be flexible and to collapse toward the outer cylindrical wall absent flow pressure in the air channel.

Optionally, the device is a source of compressed gas and wherein the air channel is configured to receive gas from the device via the connector and expel the gas through an opening between the inner cylindrical wall and the outer cylindrical wall.

Optionally, the prong is configured to provide flow communication from the opening between the inner cylindrical wall and the outer cylindrical wall to the device based on applying a vacuum through the connector.

Optionally, the nasal includes a second connector connected to a port in the inner wall, wherein the second connector is configured to provide flow communication between the bore and a sensor for sensing a parameter of breathing.

Optionally, the nasal cannula includes a pair of the prong, wherein the base connects the pair.

Optionally, the nasal cannula includes a pair of the connector, wherein each connector of the pair is fluidly connected to the air channel of one of the pair of the prong.

According to an aspect of some example embodiments, there is provided a method for providing respiratory assistance, the method comprising: providing a nasal cannula, the nasal cannula comprising: a prong formed from an inner cylindrical wall surrounded by an outer cylindrical wall, a base connecting the inner cylindrical wall to the outer cylindrical wall and an air channel formed between the inner and outer cylindrical wall, wherein the inner cylindrical wall defines a bore through which air may freely flow; and a first connector fluidly connected to the air channel and configured to provide flow communication between the air channel and an external device connected to the connector; a second connector connected to a port in the inner wall, wherein the second connector is configured to provide flow communication between the bore and a sensor for sensing a parameter of breathing; delivering air to the nasal cannula via the connector; and sensing the parameter of breathing based on connecting the second connector to the sensing device.

Optionally, the method includes extracting air from the second connectors and sensing the parameter of breathing based on the air extracted.

Optionally, the delivering and the extracting is performed concurrently.

Optionally, the delivering and the extracting is performed consecutively.

Optionally, the extracting is synchronized to occur at a defined period in a breathing cycle.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRA WING(S)

Some embodiments of the invention are herein described, by way of example only, with reference to the accompanying drawings (including an image). With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a perspective view of an example nasal cannula in accordance with some example embodiments of the present disclosure;
FIG. 2 is a top view of an example nasal cannula in accordance with some example embodiments of the present disclosure;
FIG. 3 is a cross-sectional view along a length of an example nasal cannula in accordance with some example embodiments of the present disclosure;
FIG. 4 is a same cross-sectional view of the example nasal cannula shown in FIG. 3 including indications of additional cross-sectional cuts;
FIG. 5 is section B-B of the example nasal cannula shown in FIG. 3;
FIG. 6 is section C-C of the example nasal cannula shown in FIG. 3;
FIGS. 7A, 7B, 7C and 7D is a perspective, side, front and top view respectively of another example nasal cannula in accordance with some example embodiments of the present disclosure;
FIG. 8 is a side view of the other example nasal cannula with arrows indicating example airflow directions in accordance with some example embodiments of the present disclosure;
FIG. 9 is a simplified block diagram of an example nasal cannula with associated sensors in accordance with some example embodiments;
FIG. 10 is an image of an infant fitted with an example nasal cannula and a graph of example measured flow both in accordance with some example embodiments of the present disclosure; and
FIGS. 11A, 11B and 11C are images of a child fitted with the example nasal cannula.

### DESCRIPTION OF SPECIFIC EMBODIMENTS OF THE INVENTION

The present invention, in some embodiments thereof, relates to a nasal cannula and, more particularly, but not exclusively, to a structure for a nasal cannula that does not obstruct the nasal passage.

A known drawback of nasal cannulae is that nasal cannulae tend to block a significant portion of the nasal passage due to the prongs that are placed in the nostrils. For neonates and children the blockage is even more significant and may reach 80% of the nasal passage. The blockage may make exhaling through the nose cumbersome and difficult. Adults may alter their natural breathing pattern and instead exhale orally. Neonates being obligate nose breathers cannot compensate by exhaling orally and therefore may not succeed in exhaling properly. Disruption of proper exhalation in the neonates may lead to end-distending pressure buildup that in turn may lead to gastrointestinal perforations. Another concern associated with pediatrics and especially neonates, regarding usage of a nasal cannula, is a risk that if the supply through the nasal cannula is terminated unexpectedly, the blockage due to the prongs may significantly impair the ability of the neonate or baby to take in air from the surrounding environment.

According to an aspect of some example embodiments, there is provided a nasal cannula that does not significantly obstruct the nasal passage. According to some example embodiments, the non-obstructive nasal cannula may be used for adults, children, infants as well as neonates and may support maintaining a natural breathing pattern. According to some example embodiments, the non-obstructive nasal cannula includes a pair of ring shape prongs, each of which define an annular air channel through which air may be delivered and a central bore through which a patient may have free flow communication with the surrounding environment. In some example embodiments, each prong is formed from a pair of substantially concentric cylindrical walls and a base that together define an air channel between the concentric cylindrical walls through which gasses may be actively delivered to the patient and/or sampled from the patient. The pair of substantially concentric cylindrical walls includes an inner wall that defines the central bore and an outer wall surrounding the inner wall. According to some example embodiments, both the inner wall and the outer wall extend from a ring shaped base. Flow communication with a gas source may be via a port formed in outer wall or on the ring shaped base and a tube connected to the port.

In some example embodiments, the outer wall is shaped as a truncated cone and the inner wall is defined to have a constant diameter with a height less than the outer wall. Optionally, a channel formed between the inner and outer wall is angled toward the inner wall. In some examples, a diameter of the outer wall distal from the base is substantially equal to a diameter of the inner wall. In this manner there is substantially no obstruction of the nasal passage due to the outer wall and annular air channel.

In some example embodiments, the inner wall may be formed from flexible material and may be configured to collapse toward the outer wall while no active flow is being delivered. Optionally, based on flow pressure during delivery of gas from gas source the inner wall may open the channel between the walls, e.g. move away from outer wall. By collapsing the inner wall while no active flow is being initiated, a diameter of the bore may be increased to further accommodate free flow communication with the surrounding environment.

The nasal cannula may be used as a respiratory assisting device, may be used as a sensing device for monitoring breathing parameters and may be used as both a respiratory assisting device and a sensing device. In some example embodiments, the nasal cannula includes a dedicated air channel through which sensing may be performed concurrently with respiratory assistance. Optionally the dedicated air channel may be fluidly connected to the central bore formed in the cannula and may sense air flow through the central bore during a breathing cycle. Alternately or additionally, sensing may be performed through an air channel through which gas may be delivered to the nasal cavity, e.g. the air channel that is fluidly connected to the ring shaped cavity formed in the nasal cannula.

Optionally, sensing with the nasal cannula may include for example extracting gases from the nasal cavity by applying a vacuum at an end of the tubing distal from the cannula and analyzing parameters of the extracted gases. Sensing may also include for example monitoring pressure changes. Pressure measurements may be monitored by connecting a sensor at the end of the tubing distal from the cannula.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

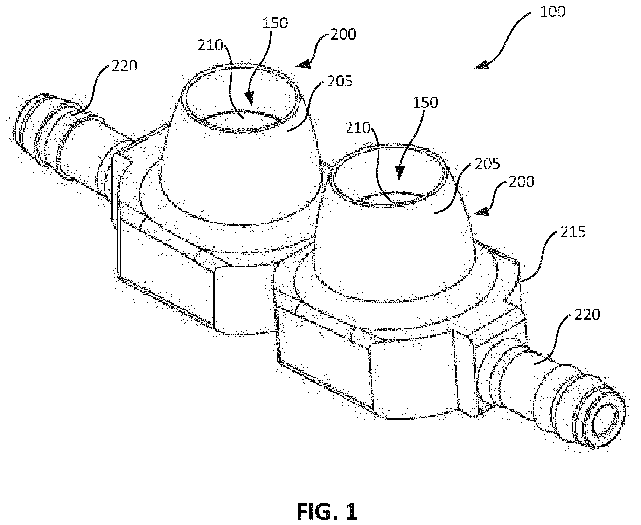

Reference is now made to FIG. 1, FIG. 2 and FIG. 3 showing a perspective, top and cross-sectional views of an example nasal cannula respectively, in accordance with some example embodiments of the present disclosure. A nasal cannula 100 may include a dedicated prong 200 for each of a right and left nostril, a dedicated connector 220 for each of a right and left nostril through which air supply is delivered to prong 200 and optionally a base 215 configured to provide structural support for prongs 200 and connectors 220.

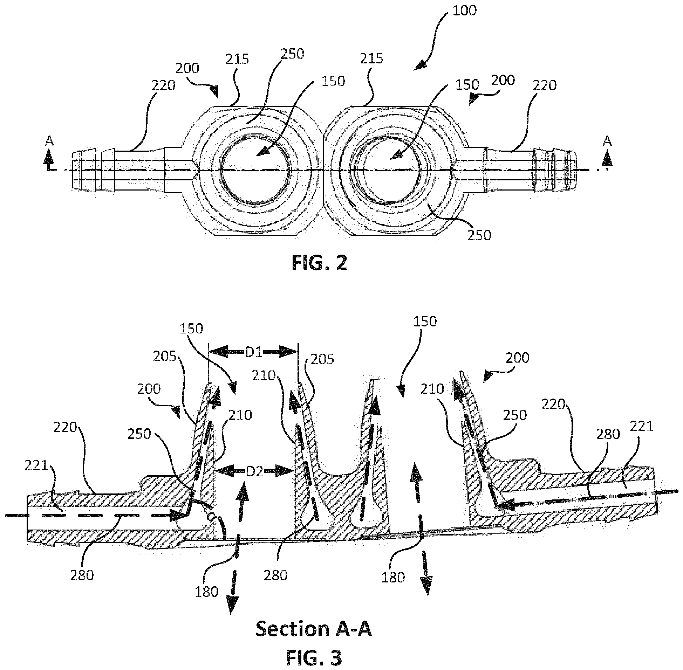

According to some example embodiments, each prong 200 includes a ring shaped cavity 250 or channel formed between an outer wall 205 and an inner wall 210 of prong 200 as well as a central bore 150 through which air may freely flow (FIG. 2 and FIG. 3). Ring shaped cavity 250 is an air channel through which air or gas may be actively delivered to nasal cavity or optionally extracted for sensing. Connector 220 may be integrated or connected to outer wall 205 and may provide flow communication between ring shaped cavity 250 and an external device. Typically, flexible tubing may be connected to connector 220 at a first end and to a device, e.g. a source of compressed air or a sensing device at an opposite end. In some example embodiments, outer wall 205 may be shaped as a truncated cone or may generally taper as it extends out from base 215. The truncated cone shape may accommodate fitting nasal cannula 100 into different size nostrils and may also reduce potentially obstruction of natural airflow as described in more detail herein below. Optionally, nasal cannula 100 may be sized to slightly expand a nostril when worn. An extent of the expansion may be based on a depth at which prongs 200 are pushed into the nostrils.

In some example embodiments, nasal cannula 100 may be formed from a polymer material. Optionally, nasal cannula 100 may be formed based on injection molding.

Referring now to FIG. 3, a diameter D2 of bore 150 may be sized to provide a relatively wide opening through which a patient may naturally breath (inhale and exhale) with airflow 180 simultaneously with receiving supplemental oxygen or increased airflow 280 with nasal cannula 100. Optionally, tapering of outer wall 205 provides a diameter D1 at the exit that is equal to diameter D2. In this manner, obstruction of natural air exchange (airflow 180) may be substantially minimized. In some example embodiments, a ratio of D2 and D1 may be defined to be 0.7 -1.0.

In some example embodiments, inner wall 210 is defined to be lower in height than a height of outer wall 205. Cavity 250 between inner wall 210 and outer wall 205 provides flow communication between a patient and a pressurized gas source connected to connectors 220. Optionally, outer wall 205 is angled toward inner wall 210. Optionally the angle of inclination α may be 60° - 85°. By angling outer wall 205 and defining inner wall 210 to be lower in height than outer wall 205, a diameter D2 of bore 150 may substantially reach diameter D1. In this manner, controlled airflow 280 may be received in the nasal cavity (or extracted from the nasal cavity) without obstructing free airflow 180 via the bore 150.

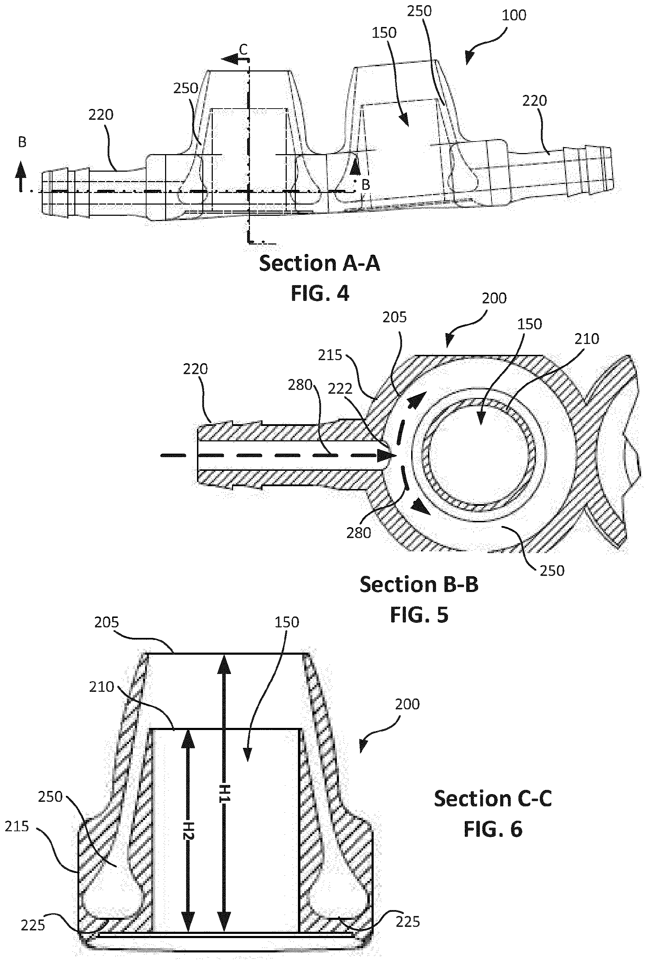

Reference is now made to FIG. 4, FIG. 5 and FIG. 6 showing additional cross-sectional views of the example nasal cannula in accordance with some example embodiments of the present disclosure. According to some example embodiments, controlled airflow 280 enters prong 200 from a lateral direction and spreads through a ring shaped cavity 250 around bore 150. Optionally, cavity 250 is a channel formed in base 215 and that extends upwards between inner wall 210 and outer wall 205. Optionally, cavity 250 may be defined by a ring shaped base 225, inner wall 210 attached to an inner diameter of the ring and outer wall 205 attached to an outer diameter of the ring, each with a sealed connection. In some example embodiments, inner wall 210 has a height H2 that is less than a height H1 of outer wall 205. Optionally a ratio H2 over H1 is defined between 0.3-1.0.

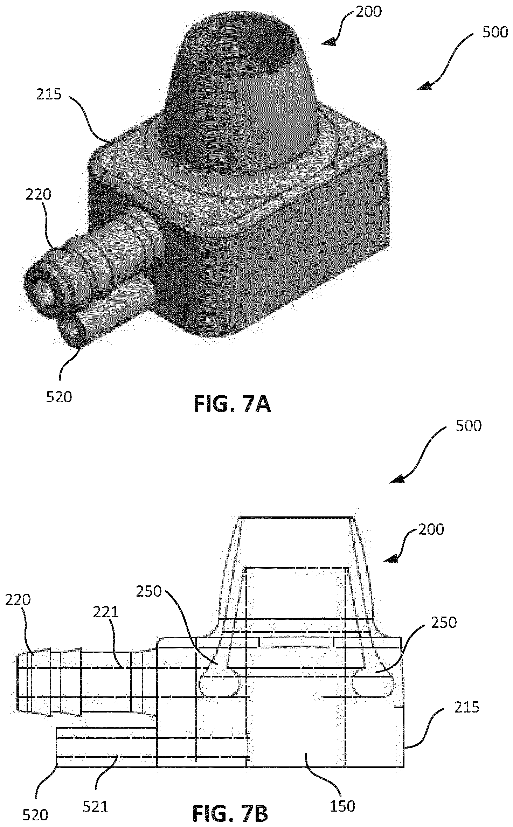

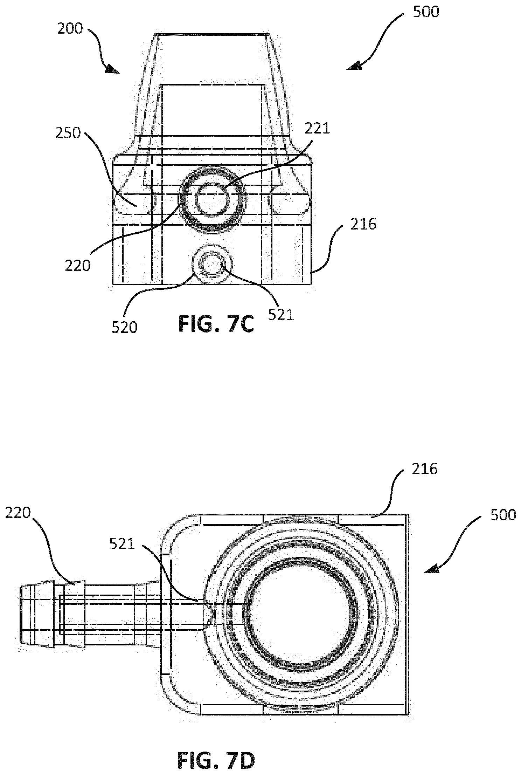

Reference is now made to FIGS. 7A, 7B, 7C and 7D showing a perspective, side, front and top view respectively of another example nasal cannula in accordance with some example embodiments of the present disclosure. According to some example embodiments, a nasal cannula 500 includes a first connector 220 defining an air flow channel 221 that is in flow communication with circumferential cavity 250 in prong 200 and may include an additional connector 520 defining an airflow channel 521 that is in fluid communication with flow through bore 150. Optionally, a base 216 provides structural support for prong 200, connector 220 and additional connector 520.

In some example embodiments, additional connector 520 may be configured to connect air flow to a sensor, e.g. pressure sensor or air sampler configured to monitor parameters while a patient is breathing. Measurements may be made while air or gas is being delivered through channel 221 into circumferential cavity 250 optionally in synchronization with a breathing rhythm and may also be made while no air or gas is being delivered. Optionally, a diameter of air flow channel 521 defined through additional connector 520 is smaller than air flow channel 221 defined through connector 220.

It is noted that although only one prong 200 is shown in nasal cannula 500 for simplicity purposes, a nasal cannula 500 may be similarly formed with a pair of prongs 200 as shown in FIGS. 1-3. Furthermore, for embodiments including a pair of prongs 200, additional connector 520 may be provided on either one or on both of prongs 200. For example, prong 200 for a left nostril may include both connectors 220 and 520 and another prong 200 for a right nostril may only include connector 220. In another example, prong 200 for a left nostril may include both connectors 220 and 520 and another prong 200 for a right nostril may also include both connectors 220 and 520. Prongs 200 for both nostrils may be formed in a single base 215 as shown for example in FIGS. 1-3.

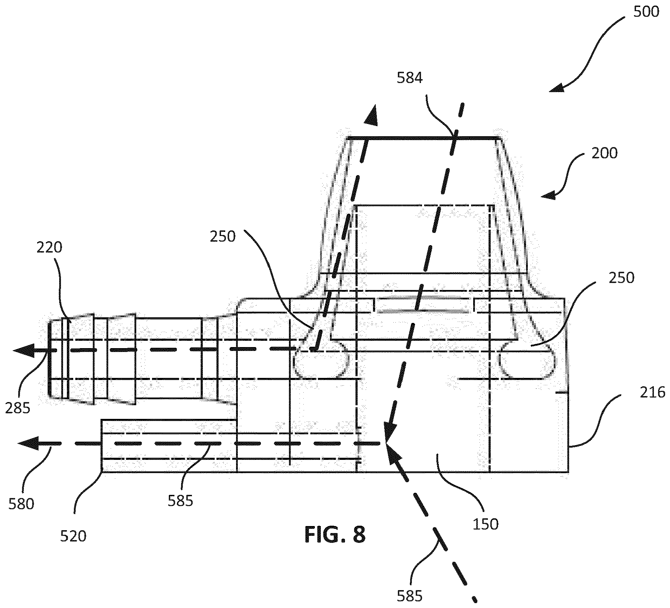

FIG. 8 is a side view of the other example nasal cannula with arrows indicating example airflow directions in accordance with some example embodiments of the present disclosure. In some example embodiments, air may be extracted from connector 520 for sensing. Optionally a vacuum may be applied to extract air from bore 150 (arrow 580). Sampled air may be air that a patient exhales (arrow 584) and may be air from the surrounding environment that a patient inhales (arrow 585). In some example embodiments, a sensor connected to connector 520 may sense parameters related to both inhalation and exhalation. In some example embodiments, sensing may be alternatively or additionally performed via connector 520.

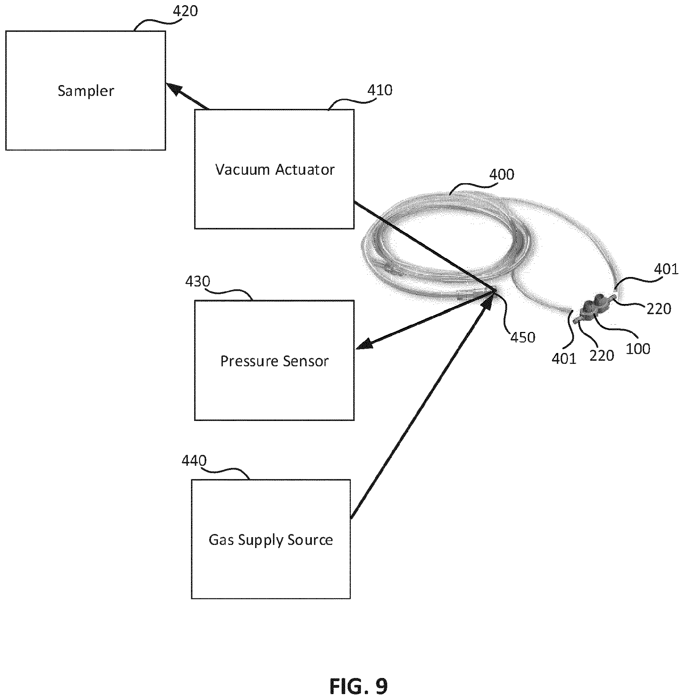

Reference is now made to FIG. 9 showing a simplified block diagram of an example nasal cannula with associated sensors in accordance with some example embodiments. According to some example embodiments, a nasal cannula 100 may be connected to tubing 400 based on attaching ends 401 to connectors 220. Tubing 400 typically includes a distal connector 450 connected to tube 400 at an end opposite an end at which nasal cannula 100 is connected. Distal connector 450 may be connected to one or more of a gas supply source 440 for delivering gas to the patient, a pressure sensor 430 configured to monitor pressure in nasal cavity during breathing, and a vacuum actuator 410, e.g. a pump for extracting air from the nasal cavity to a sampler 420 for sampling the air in the nasal cavity.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following example.

### EXAMPLE

Reference is now made to the following examples, which together with the above descriptions illustrate some embodiments of the invention in a non limiting fashion.

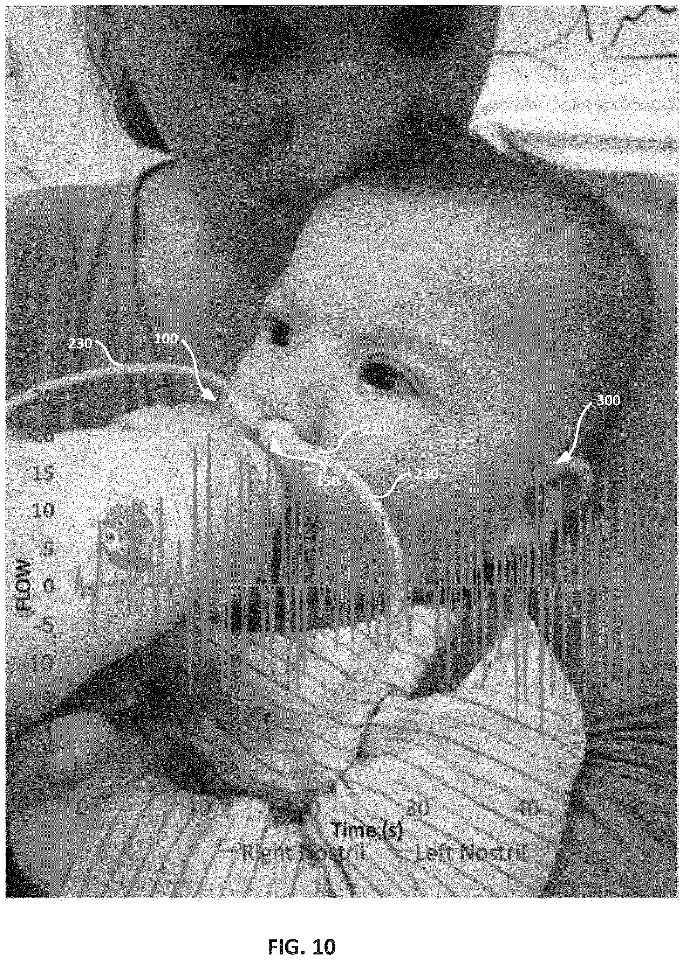

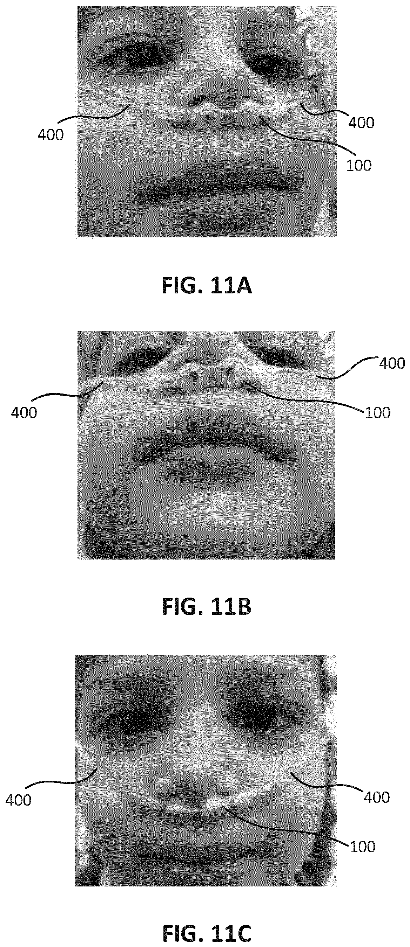

Reference is now made to FIG. 10 showing an image of an infant fitted with an example nasal cannula and to FIGS. 11A, 11B and 11C showing images of a child fitted with the example nasal cannula, both in accordance with some example embodiments of the present disclosure and a graph of example measured flow with the example nasal cannula. As can be seen in the image of FIG. 10, example nasal cannula 100 may be sized to fit an infant. While the infant may receive air or gasses delivered to nasal cannula 100 via tubes 230 and connectors 220, the infant may also freely inhale and exhale air naturally (from the surrounding environment) through bore 150 in nasal cannula 100. Graph 300 is an example graph of actual nasal airflow recorded through nasal cannula 100. The blue curve represents measurements taken from the right nostril and the orange curve represents measurements taken from the left nostril. These measurements indicate normative breathing. By providing a volume through which the infant may exhale freely, nasal cannula 100 may avoid end-distending pressure buildup and thereby the risk of gastrointestinal perforations.

Although the invention has been described in conjunction with specific embodiments thereof, it is evident that many alternatives, modifications and variations will be apparent to those skilled in the art. Accordingly, it is intended to embrace all such alternatives, modifications and variations that fall within scope of the appended claims

## Claims

1. A nasal cannula (100) comprising:
a prong (200) formed from an inner cylindrical wall (210) surrounded by an outer cylindrical wall (205) and a base (215) connecting the inner cylindrical wall (210) to the outer cylindrical wall (205), wherein the inner cylindrical wall (210), the outer cylindrical wall (205) and the base (215) together define an air channel (250) between the inner cylindrical wall (210) and the outer cylindrical wall (205), wherein the inner cylindrical wall (210), the outer cylindrical wall (205) and the air channel (250) there between are configured to be received within a nostril and wherein the inner cylindrical wall (210) defines a bore (150) through which air may freely flow; and
a connector (220) fluidly connected to the air channel (250) and configured to provide flow communication between the air channel (250) and an external device connected to the connector (220).

2. The nasal cannula (100) of claim 1, wherein the inner cylindrical wall (210) has a first height and wherein the outer cylindrical wall (205) has a second height and wherein the first height is less than the second height.

3. The nasal cannula (100) of claim 1 or claim 2, wherein the outer cylindrical (205) wall has a truncated cone shape.

4. The nasal cannula (100) of any one of claims 1-3, wherein the inner cylindrical wall (210) has a first diameter and wherein the outer cylindrical wall (205) has a second diameter at its distal end with respect to the base (215) and wherein a ratio of the first diameter over the second diameter is 0.8-1.0.

5. The nasal cannula (100) of any one of claims 1-4, wherein a width of the air channel (250) tapers distal to the base (215).

6. The nasal cannula (100) of any one of claims 1-5, wherein the connector (220) is connected to the air channel (250) via a port formed in the outer cylindrical wall.

7. The nasal cannula (100) of any one of claims 1-6, wherein the base (215) is configured to structurally support the prong (200) and the connector (220) and wherein the bore (150) defined by the inner cylindrical wall (210) extends through the base (215).

8. The nasal cannula (100) of any one of claims 1-7, wherein the inner cylindrical wall (210) is configured to be flexible and to collapse toward the outer cylindrical wall (205) absent flow pressure in the air channel (250).

9. The nasal cannula (100) of any one of claims 1-8, wherein the external device is a source of compressed gas and wherein the air channel (250) is configured to receive gas from the external device via the connector (220) and expel the gas through an opening between the inner cylindrical wall and the outer cylindrical wall.

10. The nasal cannula (100) of any one of claims 1-9, wherein the prong (200) is configured to provide flow communication from the opening between the inner cylindrical wall and the outer cylindrical wall to the external device based on applying a vacuum through the connector (220).

11. The nasal cannula (100) of any one of claims 1-10 comprising a second connector (520) connected to a port in the inner cylindrical wall, wherein the second connector (520) is configured to provide flow communication between the bore (150) and a sensor for sensing a parameter of breathing.

12. The nasal cannula (100) of any one of claims 1-11 comprising a pair of the prong (200), wherein the base (215) connects the pair.

13. The nasal cannula (100) of claim 12, comprising a pair of the connector (220), wherein each connector (220) of the pair is fluidly connected to the air channel (250) of one of the pair of the prong (200).

## Patentansprüche

1. Nasenkanüle (100), umfassend:
einen Stutzen (200), der aus einer inneren zylindrischen Wand (210), die von einer äußeren zylindrischen Wand (205) umgeben ist, gebildet ist, und eine Basis (215), die die innere zylindrische Wand (210) mit der äußeren zylindrischen Wand (205) verbindet, wobei die innere zylindrische Wand (210), die äußere zylindrische Wand (205) und die Basis (215) zusammen einen Luftkanal (250) zwischen der inneren zylindrischen Wand (210) und der äußeren zylindrischen Wand (205) definieren, wobei die innere zylindrische Wand (210 ), die äußere zylindrische Wand (205) und der Luftkanal (250) dazwischen so ausgestaltet sind, dass sie innerhalb eines Nasenlochs aufgenommen werden können, und wobei die innere zylindrische Wand (210) eine Bohrung (150) definiert, durch die Luft frei strömen kann; und
einen Verbinder (220), der mit dem Luftkanal (250) in Fluidverbindung steht und so ausgestaltet ist, dass er eine Strömungsverbindung zwischen dem Luftkanal (250) und einer externen Vorrichtung bereitstellt, die mit dem Verbinder (220) verbunden ist.

2. Nasenkanüle (100) nach Anspruch 1, wobei die innere zylindrische Wand (210) eine erste Höhe aufweist und wobei die äußere zylindrische Wand (205) eine zweite Höhe aufweist, und wobei die erste Höhe kleiner als die zweite Höhe ist.

3. Nasenkanüle (100) nach Anspruch 1 oder Anspruch 2, wobei die äußere zylindrische Wand (205) eine Kegelstumpfform aufweist.

4. Nasenkanüle (100) nach einem der Ansprüche 1-3, wobei die innere zylindrische Wand (210) einen ersten Durchmesser aufweist und wobei die äußere zylindrische Wand (205) einen zweiten Durchmesser an ihrem distalen Ende in Bezug auf die Basis (215) aufweist, und wobei ein Verhältnis des ersten Durchmessers zu dem zweiten Durchmesser 0,8-1,0 beträgt.

5. Nasenkanüle (100) nach einem der Ansprüche 1-4, wobei sich eine Breite des Luftkanals (250) distal zur Basis (215) verjüngt.

6. Nasenkanüle (100) nach einem der Ansprüche 1-5, wobei der Verbinder (220) mittels eines in der äußeren zylindrischen Wand gebildeten Ports mit dem Luftkanal (250) verbunden ist.

7. Nasenkanüle (100) nach einem der Ansprüche 1-6, wobei die Basis (215) so ausgestaltet ist, dass sie den Stutzen (200) und den Verbinder (220) strukturell trägt, und wobei sich die durch die innere zylindrische Wand (210) definierte Bohrung (150) durch die Basis (215) erstreckt.

8. Nasenkanüle (100) nach einem der Ansprüche 1-7, wobei die innere zylindrische Wand (210) so ausgestaltet ist, dass sie flexibel ist, und bei fehlendem Strömungsdruck im Luftkanal (250) in Richtung der äußeren zylindrischen Wand (205) zusammenfällt.

9. Nasenkanüle (100) nach einem der Ansprüche 1-8, wobei die externe Vorrichtung eine Quelle für Druckgas ist, und wobei der Luftkanal (250) so ausgestaltet ist, dass er Gas von der externen Vorrichtung mittels des Verbinders (220) aufnimmt und das Gas durch eine Öffnung zwischen der inneren zylindrischen Wand und der äußeren zylindrischen Wand ausstößt.

10. Nasenkanüle (100) nach einem der Ansprüche 1-9, wobei der Stutzen (200) so ausgestaltet ist, dass er eine Strömungsverbindung von der Öffnung zwischen der inneren zylindrischen Wand und der äußeren zylindrischen Wand zur externen Vorrichtung basierend auf dem Anlegen eines Vakuums durch den Verbinder (220) hindurch bereitstellt.

11. Nasenkanüle (100) nach einem der Ansprüche 1-10, die einen zweiten Verbinder (520) umfasst, der mit einem Port in der inneren zylindrischen Wand verbunden ist, wobei der zweite Verbinder (520) so ausgestaltet ist, dass er eine Strömungsverbindung zwischen der Bohrung (150) und einen Sensor zum Abfühlen eines Atemparameters bereitstellt.

12. Nasenkanüle (100) nach einem der Ansprüche 1-11, die ein Paar von Stutzen (200) umfasst, wobei die Basis (215) das Paar verbindet.

13. Nasenkanüle (100) nach Anspruch 12, die ein Paar von Verbindern (220) umfasst, wobei jeder Verbinder (220) des Paars mit dem Luftkanal (250) eines des Paares der Stutzen (200) in Fluidverbindung steht.

## Revendications

1. Canule nasale (100) comprenant :
une broche (200) formée à partir d'une paroi cylindrique intérieure (210) entourée d'une paroi cylindrique extérieure (205) et d'une base (215) reliant la paroi cylindrique intérieure (210) à la paroi cylindrique extérieure (205), dans laquelle la paroi cylindrique intérieure (210), la paroi cylindrique extérieure (205) et la base (215) définissent ensemble un canal d'air (250) entre la paroi cylindrique intérieure (210) et la paroi cylindrique extérieure (205), dans laquelle la paroi cylindrique intérieure (210), la paroi cylindrique extérieure (205) et le canal d'air (250) entre elles sont conçus pour être logés à l'intérieur d'une narine et dans laquelle la paroi cylindrique intérieure (210) définit un trou (150) à travers lequel de l'air peut circuler librement ; et
un connecteur (220) en communication fluidique avec le canal d'air (250) et conçu pour assurer une communication d'écoulement entre le canal d'air (250) et un dispositif externe relié au connecteur (220).

2. Canule nasale (100) selon la revendication 1, dans laquelle la paroi cylindrique intérieure (210) a une première hauteur et dans laquelle la paroi cylindrique extérieure (205) a une seconde hauteur et dans laquelle la première hauteur est inférieure à la seconde hauteur.

3. Canule nasale (100) selon la revendication 1 ou la revendication 2, dans laquelle la paroi cylindrique extérieure (205) a une forme de cône tronqué.

4. Canule nasale (100) selon l'une quelconque des revendications 1 à 3, dans laquelle la paroi cylindrique intérieure (210) a un premier diamètre et dans laquelle la paroi cylindrique extérieure (205) a un second diamètre à son extrémité distale par rapport à la base (215) et dans laquelle le rapport du premier diamètre au second diamètre est de 0,8 à 1,0.

5. Canule nasale (100) selon l'une quelconque des revendications 1 à 4, dans laquelle une largeur du canal d'air (250) se rétrécit de manière distale par rapport à la base (215).

6. Canule nasale (100) selon l'une quelconque des revendications 1 à 5, dans laquelle le connecteur (220) est relié au canal d'air (250) par un orifice formé dans la paroi cylindrique extérieure.

7. Canule nasale (100) selon l'une quelconque des revendications 1 à 6, dans laquelle la base (215) est conçue pour supporter structurellement la broche (200) et le connecteur (220) et dans laquelle le trou (150) défini par la paroi cylindrique intérieure (210) s'étend à travers la base (215).

8. Canule nasale (100) selon l'une quelconque des revendications 1 à 7, dans laquelle la paroi cylindrique intérieure (210) est conçue pour être flexible et pour s'affaisser en direction de la paroi cylindrique extérieure (205) en l'absence de pression d'écoulement dans le canal d'air (250).

9. Canule nasale (100) selon l'une quelconque des revendications 1 à 8, dans laquelle le dispositif externe est une source de gaz comprimé et dans laquelle le canal d'air (250) est conçu pour recevoir du gaz provenant du dispositif externe par le connecteur (220) et expulser le gaz à travers une ouverture entre la paroi cylindrique intérieure et la paroi cylindrique extérieure.

10. Canule nasale (100) selon l'une quelconque des revendications 1 à 9, dans laquelle la broche (200) est conçue pour assurer une communication d'écoulement depuis l'ouverture entre la paroi cylindrique intérieure et la paroi cylindrique extérieure jusqu'au dispositif externe sur la base de l'application d'un vide à travers le connecteur (220).

11. Canule nasale (100) selon l'une quelconque des revendications 1 à 10, comprenant un second connecteur (520) relié à un orifice dans la paroi cylindrique intérieure, dans laquelle le second connecteur (520) est conçu pour assurer une communication d'écoulement entre le trou (150) et un capteur destiné à détecter un paramètre de respiration.

12. Canule nasale (100) selon l'une quelconque des revendications 1 à 11, comprenant une paire de broches (200), dans laquelle la base (215) relie la paire.

13. Canule nasale (100) selon la revendication 12, comprenant une paire de connecteurs (220), dans laquelle chaque connecteur (220) de la paire est en communication fluidique avec le canal d'air (250) de l'une des broches de la paire de broches (200).
